# EUROPEAN PATENT SPECIFICATION

(11) **EP 3 060 302 B1**
(45) Date of publication and mention of the grant of the patent: **25.11.2020**
(21) Application number: 14841327.1
(22) Date of filing: 24.10.2014
(51) Int. Cl.: A61N 5/10

(54) **SYSTEM FOR TRIGGERING AN IMAGING PROCESS**
SYSTEM ZUM AUSLÖSEN EINES BILDGEBUNGSVERFAHRENS
SYSTÈME POUR DÉCLENCHER UN PROCESSUS D'IMAGERIE

(30) Priority: 24.10.2013 US 201314062511
(43) Date of publication of application: 31.08.2016
(73) Proprietor: Varian Medical Systems International AG, 6312 Steinhausen (CH)
(72) Inventor: MUNRO, Peter, N., 5405 Baden-Daettwil (CH); GRIMM, Stefan, 8952 Schlieren (CH); ZWIKER, Raphael, 8115 Huttikon (CH); SIDLER, Christof, 5426 Lengnau (CH)
(74) Representative: Foster, Mark Charles
(86) International application number: PCT/IB2014/002963
(87) International publication number: WO 2015/059576

(56) References cited:
- WO-A1-2011/021410
- WO-A2-2005/032647
- US-A1- 2005 058 237
- US-A1- 2012 076 270

## Description

### FIELD

This application relates to a system for triggering an imaging process.

### BACKGROUND

Radiation therapy has been employed to treat tumorous tissue. In radiation therapy, a high energy beam is applied from an external source towards the patient. The external source, which may be rotating (as in the case for arc therapy), produces a collimated beam of radiation that is directed into the patient to the target site. The dose and placement of the dose must be accurately controlled to ensure that the tumor receives sufficient radiation, and that damage to the surrounding healthy tissue is minimized.

In current radiation therapy techniques, the internal target region is periodically imaged (e.g., using x-ray) to verify the position of the internal target region during a treatment session. Applicant determines that imaging of internal target region increases radiation dose delivered to the patient. Thus, Applicant determines that it would be desirable to provide a new technique to control an initiation of an imaging process.

US 2005/058237 discloses a trigger control for controlling a number of lines of image data that can be read out from an image detector.

US2012/0076270A1 describes a method of minimizing radiation toxicity in image guided radiotherapy (IGRT) that includes using a probabilistic prediction algorithm.

### SUMMARY

The present invention provides a system for triggering an imaging process as defined in claim 1. Optional features of the system are defined in the dependent claims.

A system for triggering an imaging process may include: a processing unit that is configured for: obtaining first information regarding a state of a device; processing the first information to determine if the state of the device satisfies a first pre-defined criterion; and generating a signal to cause an imaging process to begin based at least in part on a result from the act of processing.

Optionally, the first information regarding the state of the device comprises one or more gantry positions, or an amount of change in gantry position.

Optionally, the first information regarding the state of the device comprises a couch position or a change in the couch position.

Optionally, the first information regarding the state of the device comprises a collimator configuration or a change in the collimator configuration.

Optionally, the first information regarding the state of the device comprises a total dose delivered, or an amount of change in dose delivered by the device.

Optionally, the first information regarding the state of the device comprises time, or an amount of change in time, associated with an operation of the device.

Optionally, the first information regarding the state of the device comprises a signal from the device, or an amount of change in the signal from the third party device.

Optionally, the first information regarding the state of the device comprises a signal from an operator of the device.

Optionally, the processing unit is further configured for: obtaining second information; and processing the second information to determine if a second pre-defined criterion is satisfied; wherein the processing unit is configured to generate the signal to cause the imaging process to begin based at least in part on the result from the act of processing the first information and a result from the act of processing the second information.

Optionally, the processing unit is further configured to receive a user input indicating a desire to begin the imaging process, wherein the processing unit is configured to generate the signal to cause the imaging process to begin when the result from the act of processing indicates that the first pre-defined criterion is satisfied and when the user input is received by the processing unit.

Optionally, the processing unit is further configured to use image from the imaging process to confirm a position of a target inside a subject's body.

A system for triggering an imaging process may include: a processing unit that is configured for: obtaining first information, the first information comprising dose information; processing the first information to determine if a first pre-defined criterion is satisfied; and generating a signal to cause an imaging process to begin based at least in part on a result from the act of processing.

Optionally, the dose information comprises a dose value, a set of dose values, or a change in dose.

Optionally, the dose information comprises a metric that represents a real time actual in-vivo dose.

Optionally, the processing unit is further configured for: obtaining second information; and processing the second information to determine if a second pre-defined criterion is satisfied; wherein the processing unit is configured to generate the signal to cause the imaging process to begin based at least in part on the result from the act of processing the first information and a result from the act of processing the second information.

Optionally, the second information is regarding a state of a device, the device being at least one of a gantry, a collimator, and a couch.

Optionally, the second information comprises timing information.

Optionally, the second information comprises fiducial position.

Optionally, the processing unit is further configured to receive a user input indicating a desire to begin the imaging process, wherein the processing unit is configured to generate the signal to cause the imaging process to begin when the result from the act of processing indicates that the first pre-defined criterion is satisfied and when the user input is received by the processing unit.

Optionally, the processing unit is further configured to use image from the imaging process to confirm a position of a target inside a subject's body.

Other and further aspects and features will be evident from reading the following detailed description of the embodiments.

### BRIEF DESCRIPTION OF THE DRAWINGS

The drawings illustrate the design and utility of embodiments, in which similar elements are referred to by common reference numerals. These drawings are not necessarily drawn to scale. In order to better appreciate how the above-recited and other advantages and objects are obtained, a more particular description of the embodiments will be rendered, which are illustrated in the accompanying drawings. These drawings depict only typical embodiments and are not therefore to be considered limiting of its scope.
FIG. 1A illustrates a radiation system that has image triggering capability;
FIG. 1B illustrates another radiation system that has image triggering capability;
**FIG. 1C** illustrates another radiation system that has image triggering capability;
**FIG. 2** illustrates a method of triggering an imaging process;
**FIG. 3** is an exemplary chart showing phase and amplitude for a periodic signal;
**FIG. 4** illustrates another method of triggering an imaging process;
**FIG. 5** illustrates another method of triggering an imaging process;
**FIG. 6** illustrates another method of triggering an imaging process; and
**FIG. 7** is a block diagram of a computer system architecture, with which embodiments described herein may be implemented.

### DESCRIPTION OF THE EMBODIMENTS

Various embodiments are described hereinafter with reference to the figures. It should be noted that the figures are not drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated.

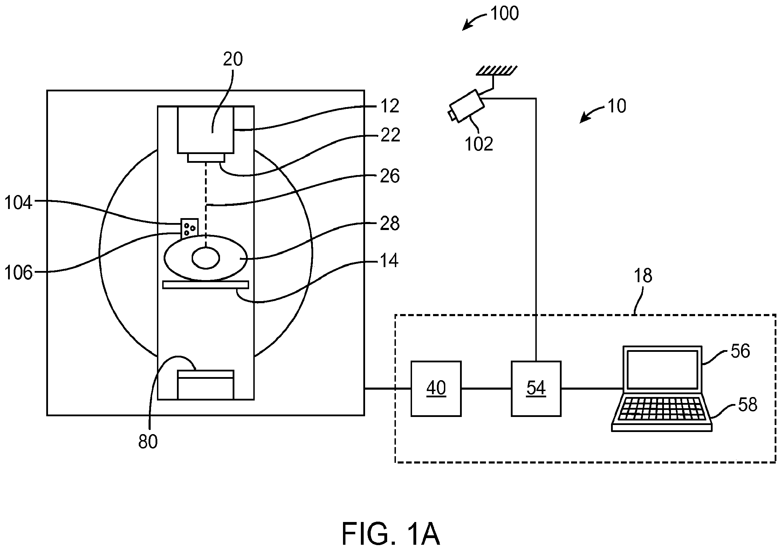

**FIG. 1A** illustrates a radiation system 10 that has image triggering capability. The system 10 is a treatment system that includes a gantry 12, a patient support 14 for supporting a patient 28, and a control system 18 for controlling an operation of the gantry 12. The gantry 12 is in a form of an arm. The system 10 also includes a radiation source 20 that projects a beam 26 of radiation towards a patient 28 while the patient 28 is supported on support 14, and a collimator system 22 for controlling a delivery of the radiation beam 26. The radiation source 20 can be configured to generate a cone beam, a fan beam, or other types of radiation beams in different embodiments.

As shown in the figure, the system 10 also includes an imager 80, located at an operative position relative to the source 20 (e.g., under the support 14). In the illustrated embodiments, the radiation source 20 is a treatment radiation source for providing treatment energy. In such cases, the treatment energy may be used to obtain images. In order to obtain imaging using treatment energies, the imager 80 is configured to generate images in response to radiation having treatment energies (e.g., MV imager). In other embodiments, in addition to being a treatment radiation source, the radiation source 20 can also be a diagnostic radiation source for providing diagnostic energy for imaging purpose. In further embodiments, the system may include the radiation source 20 for providing treatment energy, and one or more other radiation sources for providing diagnostic energy. In some embodiments, the treatment energy is generally those energies of 160 kilo-electron-volts (keV) or greater, and more typically 1 mega-electron-volts (MeV) or greater, and diagnostic energy is generally those energies below the high energy range, and more typically below 160 keV. In other embodiments, the treatment energy and the diagnostic energy can have other energy levels, and refer to energies that are used for treatment and diagnostic purposes, respectively. In some embodiments, the radiation source 20 is able to generate X-ray radiation at a plurality of photon energy levels within a range anywhere between approximately 10 keV and approximately 20 MeV.

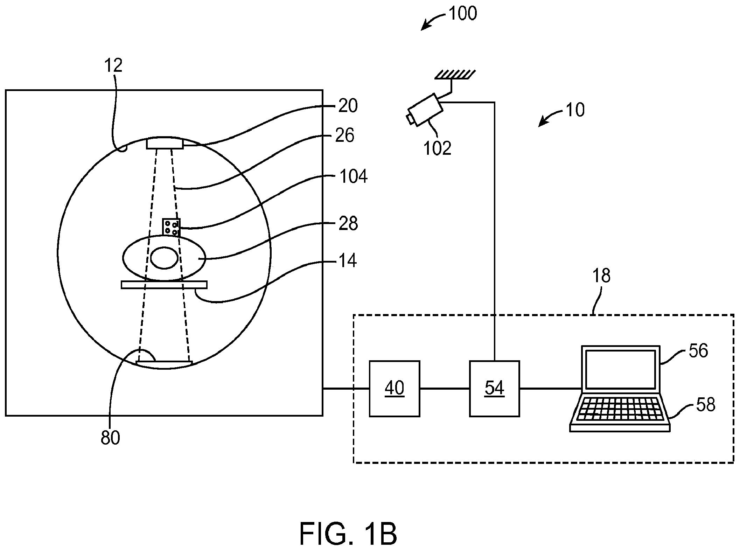

In the illustrated embodiments, the radiation source 20 is coupled to the arm gantry 12. Alternatively, the radiation source 20 may be coupled to a ring gantry (**FIG. 1B**).

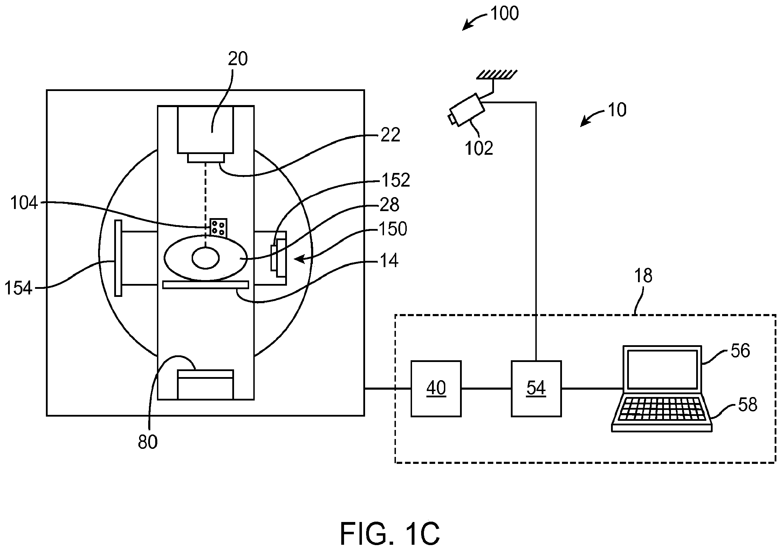

Also, in other embodiments, instead of, or in addition to, using the imager 80, a separate imaging system may be used to generate the image(s) of the internal region. For example, the separate imaging system may be a CT system, an x-ray system, an ultrasound imaging device, a MRI system, a tomosynthesis imaging system, a PET system, a SPECT system, or any other system that is capable of obtaining an image of the internal target region. In some embodiments, the separate imaging system may be coupled to the radiation system 10. For example, in some embodiments, the imaging may be provided by an imaging system 150 that has a radiation source 152, and a corresponding imager 154, like that shown in **FIG. 1C****.** In other embodiments, the separate imaging system may include one or more x-ray sources that are coupled to the gantry 12. If two x-ray sources are provided, they may be coupled to the gantry 12 at 90° relative to each other. Alternatively, they the sources may be coupled to a gantry that is moveable independent of the gantry 12. The x-ray sources may be radiation sources configured to deliver kV beams to obtain kV images. The radiation sources may be integrated into a same gantry 12 (e.g., oriented relative to each other at 90° or at other angles), or alternatively, may be separate devices that are placed adjacent to each other. In other embodiments, instead of coupling the radiation sources to the arm gantry 12, the radiation sources may be coupled to a common ring gantry, or to different respective ring gantries that can be rotated together or relative to each other.

Returning to **FIG. 1A**, in the illustrated embodiments, the control system 18 includes a processing unit 54, such as a computer processor, coupled to a control 40. The control system 18 may also include a monitor 56 for displaying data and an input device 58, such as a keyboard or a mouse, for inputting data. The operation of the radiation source 20 and the gantry 12 are controlled by the control 40, which provides power and timing signals to the radiation source 20, and controls a rotational speed and position of the gantry 12, based on signals received from the processor 54. Although the control 40 is shown as a separate component from the gantry 12 and the processor 54, in alternative embodiments, the control 40 can be a part of the gantry 12 or the processing unit 54.

As shown in the figure, the system 10 may also include a patient position monitoring system 100 that comprises a camera 102 and a marker block 104. The marker block 104 includes a plurality of markers 106, and is configured for coupling to the patient 28 during use. For example, the marker block 104 may be placed on a patient's chest, so that the marker block 104 will move correspondingly with the patient's breathing. The camera 102 is configured to view the marker block 104, and capture images of the marker block 104. The images of the marker block 104 may be processed by the processing unit 54 to determine a position of the marker block 104. The position of the marker block 104 may, in turn, be used by the processing unit 54 to determine a breathing amplitude and/or breathing phase of the patient 28.

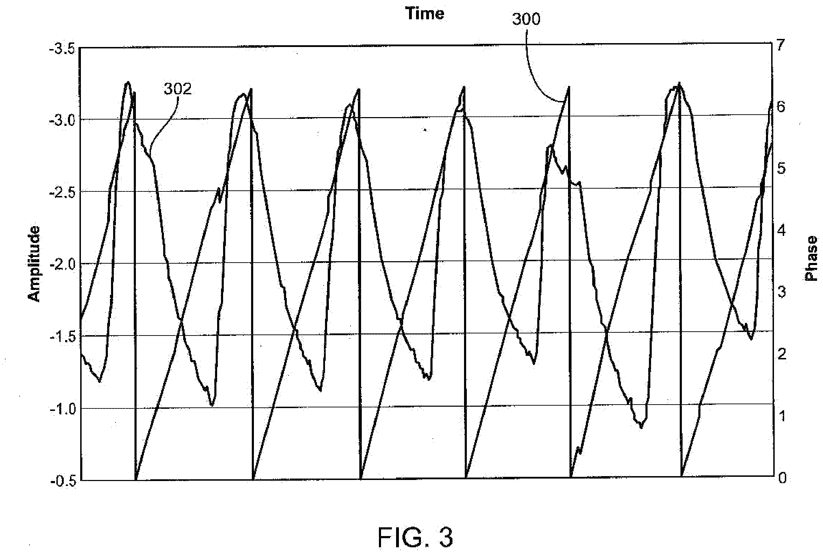

In one implementation, for each breathing amplitude (which may be a position of any bodily part that moves due to breathing, a position of an object coupled to such bodily part, or any signal that is associated with breathing), the processing unit 54 determines a corresponding breathing phase for the breathing amplitude. The phase of a physiological cycle represents a degree of completeness of a physiological cycle. In some embodiments, the phases of a respiratory cycle may be represented by a phase variable having values between 0° and 360°. **FIG. 3** illustrates an example of a phase diagram 300 that is aligned with a corresponding amplitude / position diagram 302. Amplitude diagram 302 includes positional points of the marker block 104 determined using embodiments of the technique described herein. Each point in the amplitude diagram 302 represents a position of the marker block 104 or a bodily part at a certain point in time. In the illustrated example, a phase value of 0° (and 360°) represents a peak of an inhale state, and the phase value varies linearly between 0° and 360° in a physiological cycle. As shown in the diagram, for each point in the amplitude diagram 302 at certain point in time, a corresponding phase value at the same point in time may be obtained. Thus, for each breathing amplitude, the processing unit 54 can determine the corresponding phase of the respiratory cycle. In some embodiments, the determined phase may be considered an example of a breathing signal.

In some embodiments, when using the system 10 of **FIG. 1A**, the radiation source 20 is rotated about the patient 28 to deliver treatment radiation from a plurality of gantry angles, for example, as in arc therapy. As treatment radiation is being delivered to the patient 28, the state of the patient 28, such as the patient's breathing states, may be monitored. In some embodiments, the processing unit 54 processes the signals from the camera 102 to determine breathing amplitudes of the patient 28, and then gates the delivery of the treatment radiation based on the amplitudes. For example, the processing unit 54 may cause the radiation source 20 to deliver radiation, or to stop a delivery of radiation, when the determined amplitude is within a prescribed amplitude range. In other embodiments, the processing unit 54 processes the signals from the camera 102 to determine respiratory phases of the patient 28, and then gates the delivery of the treatment radiation based on the respiratory phases. For example, the processing unit 54 may cause the radiation source 20 to deliver radiation, or to stop a delivery of radiation, when the determined phase is within a prescribed phase range. In further embodiments, the processing unit 54 processes the signals from the camera 102 to detect non-periodicity, and then gates the delivery of the treatment radiation based on the detection of non-periodicity. In other embodiments, instead of, or in addition to, controlling the delivery of radiation, the processing unit 54 may be configured to control the gantry 12 (e.g., stop, accelerate, or decelerate the gantry 12), and/or to position the patient support 14, based on the determined amplitude and/or phase, or detection of non-periodicity.

During the treatment process, the processing unit 54 monitors the patient's 28 breathing, and correlates feature(s) of the breathing (such as breathing signals, breathing amplitudes, breathing phases, etc.) with positions of internal target region that is being irradiated by the radiation beam 26. For example, based on images received from the camera 102, the processing unit 54 then determines the phase/amplitude of the breathing cycle. The phase of the breathing cycle or the amplitude is then used by the processing unit 54 to determine a position of the internal target region based on a pre-established relationship between breathing phase/amplitude and position of internal target region. In some embodiments, the relationship between the breathing phase/amplitude and target position may be pre-determined by a physician during a treatment planning process. For example, during a treatment planning process, it may be determined that when a patient is at breathing phase = 40°, the corresponding position of the internal target region is at position X=45mm, Y=23mm, and Z=6mm relative to the isocenter. This technique allows the treatment radiation system 10 to target delivery of radiation towards the target region based on breathing signals obtained by the system 10.

In the above embodiments, the system 10 is described as having a camera 102 for obtaining image signals that can be used to determine breathing amplitudes. In other embodiments, the system 10 may not include the camera 102. Instead, the system 10 may include other types of devices for providing breathing information or positional information regarding a portion of the patient 28. For example, in other embodiments, the system 10 may include a strain-gauge that is coupled to the patient 28. In such cases, the strain-gauge is communicatively coupled to the processing unit 54 for providing signals that represent breathing amplitudes of the patient 28. In other embodiments, the system 10 may include a sensor coupled to the patient's mouth and/or nose for sensing the breathing of the patient. The processing unit 54 is communicatively coupled to the sensor, and receives signals from the sensor. The signals may represent the breathing amplitudes, or may be used to obtain breathing amplitudes and/or breathing phases. In some embodiments, the breathing signal includes the position coordinates of internal anatomy landmarks, the tumor, and/or implanted fiducials. These position coordinates may be measured by various methods, including X-ray imaging, MRI, or other types of imaging. In further embodiments, internal target tracking may be employed that uses radio frequency transponder(s) implanted in or near the target region (e.g., tumor). The transponder(s) is localized by an external array antenna transmitting query signals and processing the transponder response signals. Other types of breathing sensing devices may be used with the processing unit 14 in other embodiments. In still further embodiments, the system 10 may not include any patient position monitoring device.

In the illustrated embodiments, during the treatment process, the processing unit 54 is also configured to generate a signal to trigger an imaging process to image the internal target region based at least in part on one or more pre-defined criterion being satisfied.

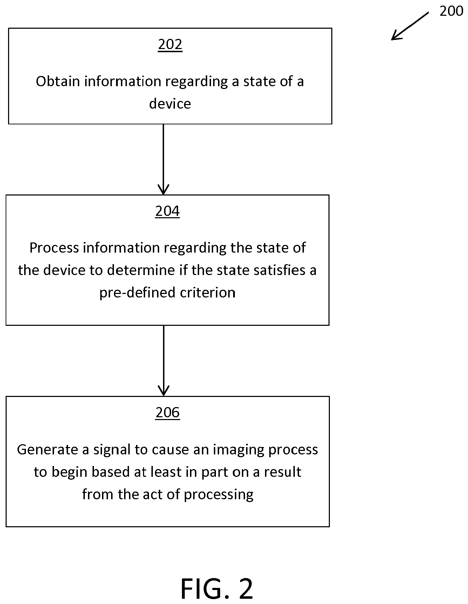

**FIG. 2** illustrates a method 200 of triggering an imaging process in accordance with some embodiments. In the method 200, the processing unit 54 obtains information regarding a state of a device (item 202).

In some embodiments, the device may be the gantry 12, and the state of the device may be one or more gantry positions, or an amount of change in the gantry position. The system 10 may include one or more sensors (e.g., position sensor(s)) for sensing a state of the gantry 12 being monitored.

In other embodiments, the device may be the collimator 22 of the system 10, and the state of the device may be a collimator configuration or a change in the collimator configuration. The system 10 may include one or more sensors for sensing a state of the collimator 22 being monitored.

In further embodiments, the device may be the patient support (couch) 14, and the state of the device may be a couch position or a change in the couch position. A couch position may be a couch position along a Z-axis, a couch position along a X-axis, a couch position along a Y-axis, a couch rotation about the X-axis, a couch rotation about the Y-axis, a couch rotation about the Z-axis, or any combination of the foregoing. In other embodiments, the device may be one or more jaws, and the state of the device may be jaw position(s). The system 10 may include one or more sensors (e.g., position and/or orientation sensor(s)) for sensing a state of the couch 14 being monitored.

In other embodiments, the device may be a radiation source, a sensor / detector for detecting an amount of dose delivered, a timer for determining an amount of time that a component has been operated (or since a last state of a component).

In still other embodiments, the device may be any component(s) that is involved in the treatment process, and may or may not be a part of the system 10. The system 10 may include one or more sensor(s) for sensing a state of the component(s) being monitored.

Returning to **FIG. 2**, next, the processing unit 54 then processes the information regarding the state of the device to determine if the state of the device satisfies a pre-defined criterion (item 204). As used in this specification, the term "pre-defined criterion" or similar term is not necessarily limited to a fixed value or values, and may refer to one or more fixed value(s), one or more variable(s) that may change (e.g., the criterion may be a function of one or more variable parameter(s)), or a combination of the foregoing.

In some embodiments, the device for which the state is being monitored may be the gantry 12, and the pre-defined criterion may be may be one or more pre-defined gantry positions, or a pre-defined amount of change in the gantry position.

In other embodiments, the device for which the state is being monitored may be the collimator 22 of the system 10, and the pre-defined criterion may be a pre-defined collimator configuration or a pre-defined change in the collimator configuration.

In further embodiments, the device for which the state is being monitored may be the patient support (couch) 14, and the pre-defined criterion may be a pre-defined couch position or a pre-defined change in the couch position.

In other embodiments, the device for which the state is being monitored may be one or more jaws, and the pre-defined criterion may be pre-defined jaw position(s).

In other embodiments, the device for which the state is being monitored may be a radiation source, and the pre-defined criterion may be pre-defined operation time, a pre-defined change in time associated with an operation of the radiation source, pre-defined amount of dose delivered, or other operation parameter(s) associated with an operation of the radiation source.

In other embodiments, the device for which the state is being monitored may be a sensor / detector for detecting an amount of dose delivered, and the pre-defined criterion may be a pre-defined total dose delivered, an pre-defined amount of change in dose delivered, etc.

In further embodiments, the device for which the state is being monitored may be a timer for determining an amount of time that a component has been operated (or since a last state of a component), and the pre-defined criterion may be a pre-defined time, or a pre-defined amount of change of time, associated with an operation of the component.

Next, the processing unit 54 generates a signal to cause an imaging process to begin based at least in part on a result from the act of processing (item 206). In particular, when the processing unit 54 determines that the state of the device satisfies the pre-defined criterion, the processing unit 54 then generates a signal to cause an image process to begin.

In one example, the device for which the state is being monitored may be the gantry 12. In such cases, the processing unit 54 generates the signal to cause the imaging process to begin when the state (e.g., one or more gantry positions, an amount of change in the gantry position, etc.) of the gantry 12 matches the pre-defined gantry state (e.g., one or more pre-defined gantry positions, a pre-defined amount of change in the gantry position, etc.). In some embodiments, the state of the gantry 12 may be considered as satisfying the pre-defined criterion if the gantry position matches a pre-defined gantry position, or if the gantry position is within a range of pre-defined gantry positions.

In another example, the device for which the state is being monitored may be a collimator 22 of the system 10. In such cases, the processing unit 54 generates the signal to cause the imaging process to begin when the state (e.g., collimator configuration, change in the collimator configuration, etc.) of the collimator 22 matches the pre-defined collimator state (e.g., a pre-defined collimator configuration, a pre-defined change in the collimator configuration, etc.).

In another example, the device for which the state is being monitored may be the patient support (couch) 14. In such cases, the processing unit 54 generates the signal to cause the imaging process to begin when the state (e.g., couch position, change in couch position, etc.) of the patient support 14 matches the pre-defined couch state (e.g., a pre-defined couch position, a pre-defined change in the couch position, etc.).

In yet another example, the device for which the state is being monitored may be one or more jaws. In such cases, the processing unit 54 generates the signal to cause the imaging process to begin when the jaw position(s) matches pre-defined jaw position(s).

In other embodiments, the device for which the state is being monitored may be a radiation source. In such cases, the processing unit 54 generates the signal to cause the imaging process to begin when a state of the radiation source matches the pre-defined state of the radiation source.

In other embodiments, the device for which the state is being monitored may be a sensor / detector for detecting an amount of dose delivered. In such cases, the processing unit 54 generates the signal to cause the imaging process to begin when and the state of the sensor / detector matches the pre-defined state of the sensor / detector.

In further embodiments, the device for which the state is being monitored may be a timer for determining an amount of time that a component has been operated (or since a last state of a component). In such cases, the processing unit 54 generates the signal to cause the imaging process to begin when the state of the timer matches the pre-defined state of the timer.

As shown in the above examples, a "state" of a device is not necessarily limited to a physical state of the device, and may refer to a physical state of the device, an operational state of the device, a performance state of the device, or a state associated with an operation of the device. Also, the device for which the state is being monitored may be a component of the radiation system, which may be provided by the supplier of the radiation system, or a third-party. In addition, in some embodiments, the information regarding the state of the device may be one or more signal(s) obtained from the device, or a change in a signal obtained from the device. In further embodiments, the information regarding the state of the device may be represented by a signal from an operator of the device. For example, in some embodiments, if an operator determines that a gantry position satisfies a certain pre-defined criterion, the operator may use an interface (e.g., a button, touch pad, etc.) to generate a signal indicating that the state of the gantry position has been satisfied. The signal is then transmitted to the processing unit 54, which determines that the state of the gantry satisfies the pre-defined criterion based on the receipt of the signal.

In the illustrated embodiments, if the radiation system 10 has imaging capability (e.g., if the radiation system 10 has the imager 80), the triggered image process may be performed using the imager 80 of the system 10. For example, the radiation source 20 may deliver imaging radiation having diagnostic energy (e.g., in kv range), or radiation having treatment energy level (e.g., in MeV range) to generate one or more images of the internal region using the imager 80. Alternatively, a separate imaging system may be used to generate the image(s) of the internal region. For example, the separate imaging system may be a CT system, an x-ray system, an ultrasound imaging device, a MRI system, a tomosynthesis imaging system, a PET system, a SPECT system, or any other system that is capable of obtaining an image of the internal target region. In some embodiments, the separate imaging system may be coupled to the radiation system 10. For example, in some embodiments, the separate imaging system may include one or more x-ray sources that are coupled to the gantry 12. If two x-ray sources are provided, they may be coupled to the gantry 12 at 90° relative to each other.

The image(s) obtained from the imaging process triggered by the signal from the processing unit 54 may be any types of image(s). For example, the image(s) may be x-ray image(s), CT image(s), ultrasound image(s), MRI image(s), tomosynthesis image(s), PET image(s), SPECT image(s), PET-CT image(s), etc. Also, in some embodiments, the image(s) may be MV image(s). In other embodiments, the image(s) may be kV image(s).

The image(s) triggered by the imaging process may be stored in a non-transitory medium in some embodiments.

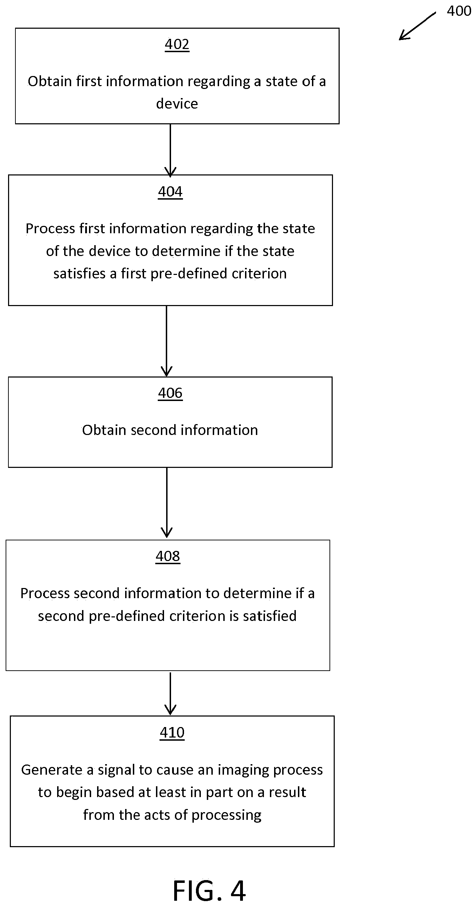

In the above embodiments, the imaging process is triggered by the signal from the processing unit 54 based on a state of a device satisfying a pre-defined criterion. In other embodiments, the generating of the signal by the processing unit 54 may be performed in response to two or more criteria being satisfied. For example, as shown in the method 400 of **FIG. 4****,** in some embodiments, the processing unit 54 may obtain first information regarding a device state (item 402), and processes the first information to determine if a first pre-defined criterion is satisfied (item 404). Items 402, 404 are similar to items 202, 204 discussed previously. As shown in the figure, the method 400 further includes obtaining second information (item 406) by the processing unit 54, and processing the second information to determine if a second pre-defined criterion is satisfied (item 408). When the processing unit 54 determines that both the first and second pre-defined criteria are satisfied, the processing unit 54 then generates a signal to cause an imaging process (item 410).

In one example, the second information may be information regarding a state of an additional device (e.g., a gantry, a collimator, a couch, jaw(s), radiation source, sensor / detector, timer, etc.). In such cases, the processing unit 54 determines if the additional device state matches with a pre-defined device state in item 408. If so, the processing unit 54 then generates the signal to trigger the imaging process in item 410. In some embodiments, the first information may be information regarding a state of the gantry 12, and the second information may be information regarding a state of the couch 14. In such cases, the system 10 may include a first set of one or more sensors for sensing a state of the gantry 12, and a second set of one or more sensors for sensing a state of the couch 14.

In another example, the second information may be timing information (e.g., how much time has lapsed since a certain event). In such cases, the processing unit 54 determines if the timing information matches with a pre-defined timing criterion in item 408. If so, the processing unit 54 then generates the signal to trigger the imaging process in item 410. In some embodiments, the system 10 may include one or more timers for determining the timing information.

In further example, the second information may be dose information (e.g., a dose value, a set of dose values, or a change in dose, etc.). The dose information may be real time in-vivo dose, or may be a metric (dose surrogate) that represents a real time actual in-vivo dose. The processing unit 54 determines if the dose information matches with a pre-defined dose criterion (e.g., a dose value or a range of dose values) in item 408. If so, the processing unit 54 then generates the signal to trigger the imaging process in item 410. In some embodiments, the system 10 may include one or more dose sensors / calculators for obtaining the dose information.

In another example, the second information may be fiducial position (e.g., a position of a marker inside a patient, a position of a marker outside a patient, a position of an organ, a position of a portion of the patient, etc.). In such cases, the processing unit 54 determines if the fiducial position matches with a pre-defined positional criterion (e.g., a pre-defined fiducial position, or a range of pre-defined fiducial positions) in item 408. If so, the processing unit 54 then generates the signal to trigger the imaging process in item 410. In some embodiments, the fiducial position may be obtained using the camera system 100, which uses the camera 102 to view the marker block 104. Alternatively, instead of using the marker block 104, the camera 102 may be configured to view one or more landmarks on a patient's body that function as fiducial(s). In other embodiments, the fiducial position may be obtained using other types of position sensing techniques. For example, in other embodiments, one or more markers may be implanted inside the patient. In such cases, a sensor located outside the patient may be configured to sense the marker(s) inside the patient. The marker(s) may be passive marker(s) which does not emit energy. Alternatively, the marker(s) may be active marker(s) that emit energy for detection by the sensor outside the patient. The energy may be RF energy, electromagnetic energy, ultrasound energy, etc. In further embodiments, an external or internal anatomy (e.g., diaphragm, spine, etc.) of the patient may be used as the fiducial, or as a surrogate of a fiducial.

It should be noted that items 402-410 in the method 400 of **FIG. 4** do not need to be performed in the order shown, and that the items 402-410 may be performed in other orders in other embodiments. For example, in other embodiments, the act of obtaining the second information in item 406, may be performed before, or simultaneously with, the act of obtaining the first information in item 402. Similarly, the act of processing the second information in item 408 may be performed before, or simultaneously with, the act of processing the first information in item 404.

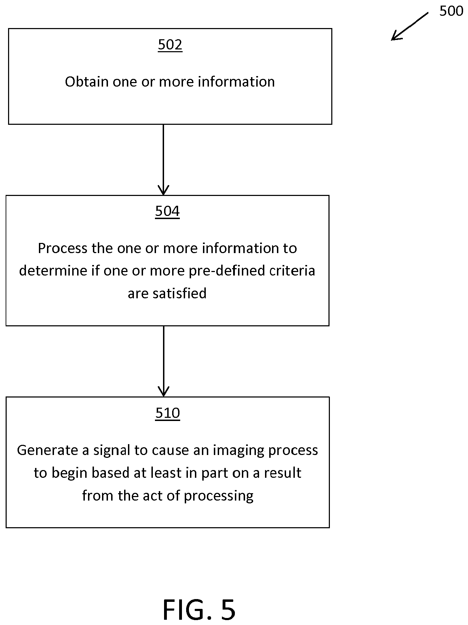

It should be noted that the technique for triggering an imaging process is not limited to being based on a state of a device satisfying a pre-defined criterion, and that other criterion / criteria may also be used. For example, as shown in the method 500 of **FIG. 5**, in other embodiments, the processing unit 54 may be configured to obtain one or more information (item 502), and process the one or more information to determine if one or more respective pre-defined criteria are satisfied (item 504). By means of non-limiting examples, the one or more information may be information regarding a state of a device, information regarding a plurality of states of respective different devices, timing information, dose information, fiducial position, or any combination thereof. Examples of these information were discussed previously with reference to the method of **FIG. 4**, and therefore, the details of such will not be repeated. If the processing unit 54 determines that one or more predefined criteria are satisfied, the processing unit 54 then generates a signal to trigger an imaging process to begin (item 510).

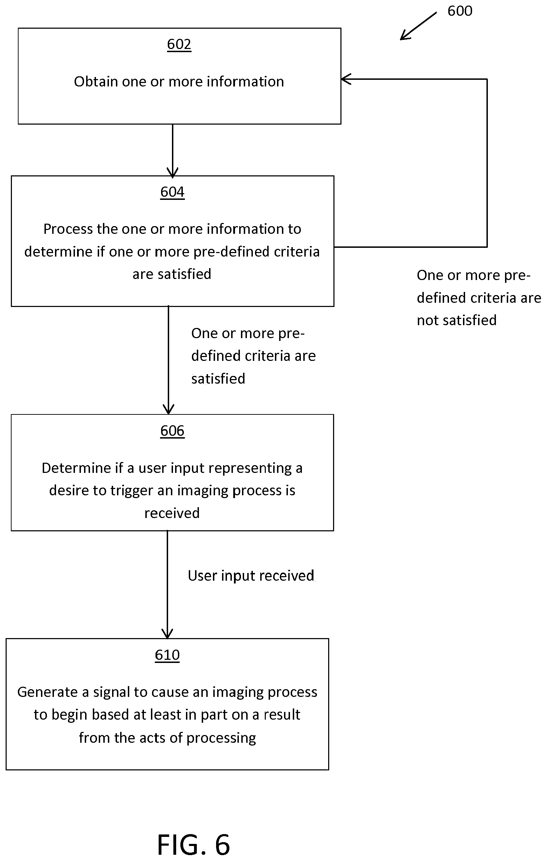

In some embodiments (such as the embodiments of **FIGS. 2**, **4**, and **5**), the processing unit 54 may be configured to automatically generate the signal to trigger the imaging process when one or more pre-defined criteria are satisfied. In such cases, no user input is need. In other embodiments, the processing unit 54 may be configured to receive a user input representing a desire to trigger an imaging process, and will generate the signal to trigger the imaging process when (1) one or more pre-defined criteria are satisfied, and (2) when the user input representing a desire to trigger an imaging process is received. In such cases, the triggering of the imaging process may be considered "semi-automatic". For example, as shown in the method 600 of **FIG. 6**, in some embodiments, the processing unit 54 may be configured to obtain one or more information (item 602), and process the one or more information to determine if one or more respective pre-defined criteria are satisfied (item 604). Items 602, 604 may be items 202, 204, or items 402, 404, 406, 408 in some embodiments. If the processing unit 54 determines that one or more predefined criteria are satisfied, the processing unit 54 then also determines whether a user input representing a desire to trigger an imaging process has been received (item 606). If so, the processing unit 54 then generates a signal to trigger an imaging process to begin (item 610).

It should be noted that items 602-606 in the method 600 of **FIG. 6** do not need to be performed in the order shown, and that the items 602-606 may be performed in other orders in other embodiments. For example, in other embodiments, the act of determining if a user input representing a desire to trigger an imaging process in item 606 may be performed before item 602 and/or item 604.

It should be noted that the user input representing the desire to trigger the imaging process may be received anytime. For example, in some embodiments, the processing unit 54 may receive the user input before the one or more information in item 602 are obtained. In such cases, the user may input a command to the processing unit 54 indicating a desire to trigger an imaging process. The processing unit 54 then obtains one or more information in item 602, and determines if one or more respective pre-defined criteria are satisfied in item 604. If so, the processing unit 54 then automatically generates a signal to trigger an imaging process to begin.

In the embodiments of **FIGS. 2**, **4**, **5**, and **6**, the image(s) obtained from the imaging process triggered by the signal from the processing unit 54 may be used for different purposes. In some embodiments, the image(s) of the internal target region is used by the processing unit 54 to verify the position of the target region, and/or to confirm the pre-established relationship between breathing feature (amplitude, phase, etc.) and target position. In other embodiments, the image of the internal target region may also be used by the processing unit 54 to verify the relationship between breathing feature and target position (e.g., external-internal correlation model). If a result of the verification process indicates that the model is inaccurate, the processing unit 54 may update (e.g., modify, recreate, etc.) the relationship between breathing feature and target position (e.g., external-internal correlation model), so that the updated relationship may be used by the system 10 to deliver additional radiation to the patient 28 (e.g., to control the radiation source 20, collimator 22, gantry 12, and/or patient support 14). In other embodiments, the processing unit 54 may cause the radiation process to stop if the result of the verification process indicates that the model is inaccurate.

In other embodiments, the image(s) of the internal target region determined from the method 200 /400 / 500 / 600 may be used by the processing unit 54 to determine the position of the internal target region using stereo imaging technique.

Also, in one or more embodiments described herein, the image(s) of the internal target region determined from the method 200 / 400 / 500 / 600 may be used by the processing unit 54 to actively track a target region (e.g., a tumor).

In addition, in one or more embodiments described herein, the image(s) of the internal target region determined from the method 200 / 400 / 500 / 600 may be used by the processing unit 54 for matching with reference image(s). In some embodiments, the matching of the automatically triggered image(s) with reference image(s) may be performed by the processing unit 54 in real time (i.e., shortly (e.g., within 5 seconds, and preferably within 3 seconds, and more preferably within 1 second) after the triggered image(s) are obtained). In some cases, the processing unit 54 may also be configured to automatically correct a patient's position based at least in part on a result from the act of matching the triggered image(s) with the reference image(s). For example, if a result of the matching indicates that the patient is offset in the X-direction by 0.3 mm, then processing unit 54 may then generate a signal to move the patient support 14 accordingly so that a target region in the patient 28 is aligned with a desired position.

Also, in one or more embodiments described herein, the processing unit 54 may process the image(s) obtained from the triggered imaging process for presentation to a user. For example, in some embodiments, the processing unit 54 may operate a display to overlay reference structures and contours (e.g., obtained from planning CT images, DRRs, etc.) onto the image(s) acquired from the triggered imaging process, or vice versa. This allows quick visual inspection of the image(s), and also assessment of the current tumor or patient position with respect to the planned position. In one implementation, the processing unit 54 may determine a gantry angle at which the triggered image is obtained. The processing unit 54 then retrieves a CT volumetric image (e.g., created from a treatment planning session, or in other medical session), and determines a two-dimensional image from the CT volumetric image with an orientation that corresponds (e.g., matches) with the gantry angle at which the triggered image is obtained. This allows the two images with the same orientation to be compared with respect to each other.

Furthermore, in one or more embodiments (e.g., the embodiment of **FIG. 2**, **4**, **5**, or **6**) described herein, in addition to triggering an imaging process in response to one or more pre-defined criteria being satisfied, the processing unit 54 may also generate one or more additional signals to operate one or more devices. For example, the processing unit 54 may generate a signal to cause the radiation source 20 to deliver radiation, or to stop a delivery of radiation. In other embodiments, instead of, or in addition to, controlling the delivery of radiation, the processing unit 54 may be configured to control the gantry 12 (e.g., stop, accelerate, or decelerate the gantry 12), operate the collimator 22, and/or to position the patient support 14.

It should be noted that using one or more device states to trigger an imaging of internal region is advantageous because it obviates the need to periodically image the internal region for verification of the position of the internal region and for verification of the relationship between breathing and target positions. Periodic imaging of internal region at constant intervals is not desirable because it complicates and lengthens the treatment procedure. Also, in the case in which radiation is used to image internal region, periodic imaging using radiation is also not desirable because it increases the radiation dosage to the patient 28.

Also, it should be note that the above embodiments (e.g., the embodiments of the methods of **FIGS. 2**, **4**, **5**, and **6**) may be performed during treatment of lung tumors, or any of other targets at other locations (such as spine, prostate, head, etc.), which may or may not be moving due to patient's physiological movements. The triggered image(s) may allow quick identification of target that has moved away from its desired position during treatment, such as respiratory gated treatment of moving anatomy (e.g., lung, liver, pancreas, etc.), un-gated treatment of moving anatomy (e.g., lung, liver, pancreas, etc.), un-gated treatment of stationary anatomy (e.g., skull, spine, prostate, etc.), etc.

### Computer System Architecture

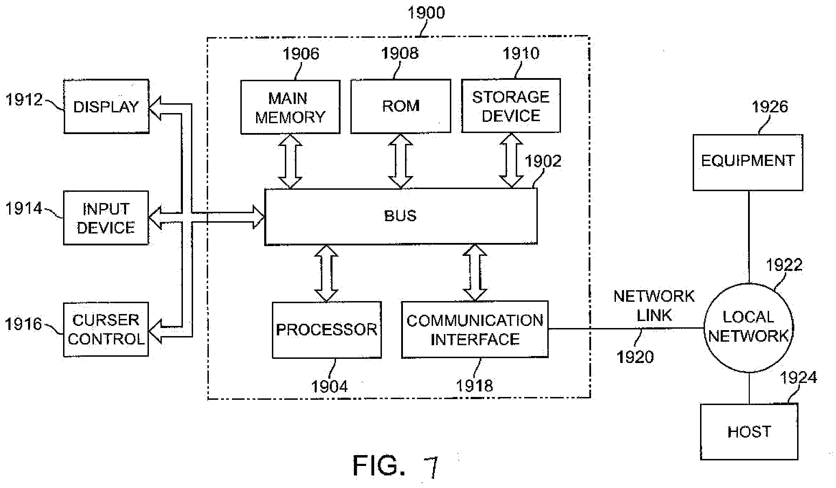

**FIG. 7** is a block diagram that illustrates an embodiment of a computer system 1900 upon which an embodiment of the invention may be implemented. Computer system 1900 includes a bus 1902 or other communication mechanism for communicating information, and a processor 1904 coupled with the bus 1902 for processing information. The processor 1904 may be an example of the processing unit 54 of **FIG. 1**, or another processor that is used to perform various functions described herein. The computer system 1900 also includes a main memory 1906, such as a random access memory (RAM) or other dynamic storage device, coupled to the bus 1902 for storing information and instructions to be executed by the processor 1904. The main memory 1906 also may be used for storing temporary variables or other intermediate information during execution of instructions to be executed by the processor 1904. The computer system 1900 further includes a read only memory (ROM) 1908 or other static storage device coupled to the bus 1902 for storing static information and instructions for the processor 1904. A data storage device 1910, such as a magnetic disk or optical disk, is provided and coupled to the bus 1902 for storing information and instructions.

The computer system 1900 may be coupled via the bus 1902 to a display 1912, such as a cathode ray tube (CRT) or a flat panel (e.g., LCD), for displaying information to a user. An input device 1914, including alphanumeric and other keys, is coupled to the bus 1902 for communicating information and command selections to processor 1904. Another type of user input device is cursor control 1916, such as a mouse, a trackball, or cursor direction keys for communicating direction information and command selections to processor 1904 and for controlling cursor movement on display 1912. This input device typically has two degrees of freedom in two axes, a first axis (e.g., x) and a second axis (e.g., y), that allows the device to specify positions in a plane.

The computer system 1900 may be used for performing various functions (e.g., calculation) in accordance with the embodiments described herein. According to one embodiment, such use is provided by computer system 1900 in response to processor 1904 executing one or more sequences of one or more instructions contained in the main memory 1906. Such instructions may be read into the main memory 1906 from another computer-readable medium, such as storage device 1910. Execution of the sequences of instructions contained in the main memory 1906 causes the processor 1904 to perform the process steps described herein. One or more processors in a multi-processing arrangement may also be employed to execute the sequences of instructions contained in the main memory 1906. In alternative embodiments, hard-wired circuitry may be used in place of or in combination with software instructions to implement the invention. Thus, embodiments of the invention are not limited to any specific combination of hardware circuitry and software.

The term "computer-readable medium" as used herein refers to any medium that participates in providing instructions to the processor 1904 for execution. Such a medium may take many forms, including but not limited to, non-volatile media, volatile media, and transmission media. Non-volatile media includes, for example, optical or magnetic disks, such as the storage device 1910. A non-volatile medium may be considered as an example of a non-transitory medium. Volatile media includes dynamic memory, such as the main memory 1906. A volatile medium may be considered as another example of a non-transitory medium. Transmission media includes coaxial cables, copper wire and fiber optics, including the wires that comprise the bus 1902. Transmission media can also take the form of acoustic or light waves, such as those generated during radio wave and infrared data communications.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, a PROM, and EPROM, a FLASH-EPROM, any other memory chip or cartridge, a carrier wave as described hereinafter, or any other medium from which a computer can read.

Various forms of computer-readable media may be involved in carrying one or more sequences of one or more instructions to the processor 1904 for execution. For example, the instructions may initially be carried on a magnetic disk of a remote computer. The remote computer can load the instructions into its dynamic memory and send the instructions over a telephone line using a modem. A modem local to the computer system 1900 can receive the data on the telephone line and use an infrared transmitter to convert the data to an infrared signal. An infrared detector coupled to the bus 1902 can receive the data carried in the infrared signal and place the data on the bus 1902. The bus 1902 carries the data to the main memory 1906, from which the processor 1904 retrieves and executes the instructions. The instructions received by the main memory 1906 may optionally be stored on the storage device 1910 either before or after execution by the processor 1904.

The computer system 1900 also includes a communication interface 1918 coupled to the bus 1902. The communication interface 1918 provides a two-way data communication coupling to a network link 1920 that is connected to a local network 1922. For example, the communication interface 1918 may be an integrated services digital network (ISDN) card or a modem to provide a data communication connection to a corresponding type of telephone line. As another example, the communication interface 1918 may be a local area network (LAN) card to provide a data communication connection to a compatible LAN. Wireless links may also be implemented. In any such implementation, the communication interface 1918 sends and receives electrical, electromagnetic or optical signals that carry data streams representing various types of information.

The network link 1920 typically provides data communication through one or more networks to other devices. For example, the network link 1920 may provide a connection through local network 1922 to a host computer 1924 or to equipment 1926 such as a radiation beam source or a switch operatively coupled to a radiation beam source. The data streams transported over the network link 1920 can comprise electrical, electromagnetic or optical signals. The signals through the various networks and the signals on the network link 1920 and through the communication interface 1918, which carry data to and from the computer system 1900, are exemplary forms of carrier waves transporting the information. The computer system 1900 can send messages and receive data, including program code, through the network(s), the network link 1920, and the communication interface 1918.

Although particular embodiments of the present inventions have been shown and described, it will be understood that it is not intended to limit the present inventions to the preferred embodiments, and it will be obvious to those skilled in the art that various changes and modifications may be made without departing from the scope of the present inventions. For example, the term "image" needs not be limited to an image that is displayed visually, and may refer to image data that is stored. Also, the term "processing unit" may include one or more processors, or may refer to any device that is capable of performing mathematical computation implemented using hardware and/or software. The term "processing unit" may also refer to software stored in a non-transitory medium in other embodiments. Further, in any of the embodiments described herein, instead of using the processing unit 54 to perform the various functions described, a separate processing unit may be used. The embodiments, aspects and examples which do not fall within the scope of the claims are provided for illustrative purpose only and do not form part of the present invention. The invention is defined in the claims.

## Claims

1. A system (10) for triggering an imaging process during a radiation treatment process, comprising:
a processing unit (54) that is configured to:
obtain first information; and
process the first information to determine if a first pre-defined criterion is satisfied (204, 404);
wherein the processing unit (54) is configured to generate a signal to cause an imaging process that includes a delivery of imaging energy to begin based at least in part on a result from the act of processing (206), wherein the first information is regarding a state of a device of the treatment process (202, 402);
**characterized in that**:
the first information comprises an amount of time that the device has been operated.

2. The system of claim 1, wherein the first information regarding the state of the device comprises one or more gantry positions, or an amount of change in gantry position.

3. The system of claim 1, wherein the first information regarding the state of the device comprises a couch position or a change in the couch position.

4. The system of claim 1, wherein the first information regarding the state of the device comprises a collimator configuration or a change in the collimator configuration.

5. The system of claim 1, wherein the first information regarding the state of the device comprises a total dose delivered, or an amount of change in dose delivered by the device.

6. The system of claim 1, wherein the first information regarding the state of the device comprises time, or an amount of change in time, associated with an operation of the device.

7. The system of claim 1, wherein the first information regarding the state of the device comprises a signal from the device, or an amount of change in the signal from the third party device.

8. The system of claim 1, wherein the first information regarding the state of the device comprises a signal from an operator of the device.

9. The system of claim 1, wherein the processing unit (54) is further configured for:
obtaining second information (406); and
processing the second information to determine if a second pre-defined criterion is satisfied (408);
wherein the processing unit (54) is configured to generate the signal to cause the imaging process to begin based at least in part on the result from the act of processing the first information and a result from the act of processing the second information (410).

10. The system of claim 9, wherein the second information comprises timing information or fiducial position.

11. The system of claim 1, wherein the device comprises at least one of a gantry (12), a collimator (22), and a couch (14).

12. The system of claim 1, wherein the processing unit (54) is further configured to receive a user input indicating a desire to begin the imaging process, wherein the processing unit (54) is configured to generate the signal to cause the imaging process to begin when the result from the act of processing indicates that the first pre-defined criterion is satisfied and when the user input is received by the processing unit (54).

13. The system of claim 1, wherein the processing unit (54) is further configured to use images from the imaging process to confirm a position of a target inside a subject's body.

14. The system of claim 1, wherein said first information comprises dose information comprising a dose value, a set of dose values, or a change in dose.

15. The system of claim 1, wherein said first information comprises dose information comprising a metric that represents a real time actual in-vivo dose.

## Patentansprüche

1. System (10) zum Auslösen eines Bildgebungsprozesses während eines Strahlenbehandlungsprozesses, umfassend:
eine Verarbeitungseinheit (54), die zu Folgendem konfiguriert ist:
Erhalten von ersten Informationen; und
Verarbeiten der ersten Informationen, um zu bestimmen, ob ein erstes vordefiniertes Kriterium erfüllt ist (204, 404) ;
wobei die Verarbeitungseinheit (54) dazu konfiguriert ist, ein Signal zu erzeugen, um zu bewirken, dass ein Bildgebungsprozess, der eine Abgabe von Bildgebungsenergie beinhaltet, beginnt, zumindest teilweise basierend auf einem Ergebnis des Vorgangs des Verarbeitens (206), wobei die ersten Informationen einen Zustand einer Vorrichtung des Behandlungsprozesses (202, 402) betreffen;
**dadurch gekennzeichnet, dass**:
die ersten Informationen eine Zeitdauer umfassen, für die die Vorrichtung betrieben wurde.

2. System nach Anspruch 1, wobei die ersten Informationen, die den Zustand der Vorrichtung betreffen, eine oder mehrerer Gantry-Positionen oder einen Betrag der Änderung der Gantry-Position umfassen.

3. System nach Anspruch 1, wobei die ersten Informationen, die den Zustand der Vorrichtung betreffen, eine Behandlungstischposition oder eine Änderung der Behandlungstischposition umfassen.

4. System nach Anspruch 1, wobei die ersten Informationen, die den Zustand der Vorrichtung betreffen, eine Kollimatorkonfiguration oder eine Änderung der Kollimatorkonfiguration umfassen.

5. System nach Anspruch 1, wobei die ersten Informationen, die den Zustand der Vorrichtung betreffen, eine abgegebene Gesamtdosis oder einen Betrag der Änderung der durch die Vorrichtung abgegebenen Dosis umfassen.

6. System nach Anspruch 1, wobei die ersten Informationen, die den Zustand der Vorrichtung betreffen, eine Zeit oder einen Betrag der Änderung der Zeit, die einem Betrieb der Vorrichtung zugeordnet ist, umfassen.

7. System nach Anspruch 1, wobei die ersten Informationen, die den Zustand der Vorrichtung betreffen, ein Signal von der Vorrichtung oder einen Betrag der Änderung des Signals von der externen Vorrichtung umfassen.

8. System nach Anspruch 1, wobei die ersten Informationen, die den Zustand der Vorrichtung betreffen, ein Signal von einem Betreiber der Vorrichtung umfassen.

9. System nach Anspruch 1, wobei die Verarbeitungseinheit (54) ferner zu Folgendem konfiguriert ist:
Erhalten von zweiten Informationen (406); und
Verarbeiten der zweiten Informationen, um zu bestimmen, ob ein zweites vordefiniertes Kriterium erfüllt ist (408);
wobei die Verarbeitungseinheit (54) dazu konfiguriert ist, das Signal zu erzeugen, um zu bewirken, dass der Bildgebungsprozess beginnt, zumindest teilweise basierend auf dem Ergebnis des Vorgangs des Verarbeitens der ersten Informationen und einem Ergebnis des Vorgangs des Verarbeitens der zweiten Informationen(410).

10. System nach Anspruch 9, wobei die zweiten Informationen Zeitinformation oder eine Bezugsposition umfassen.

11. System nach Anspruch 1, wobei die Vorrichtung mindestens eines von einer Gantry (12), einem Kollimator (22), und einem Behandlungstisch (14) umfasst.

12. System nach Anspruch 1, wobei die Verarbeitungseinheit (54) ferner dazu konfiguriert ist, eine Benutzereingabe zu empfangen, die einen Wunsch anzeigt, den Bildgebungsprozess zu beginnen, wobei die Verarbeitungseinheit (54) dazu konfiguriert ist, das Signal zu erzeugen, um zu bewirken, dass der Bildgebungsprozess beginnt, wenn das Ergebnis des Vorgangs des Verarbeitens anzeigt, dass das erste vordefinierte Kriterium erfüllt ist und wenn die Benutzereingabe von der Verarbeitungseinheit (54) empfangen wird.

13. System nach Anspruch 1, wobei die Verarbeitungseinheit (54) ferner dazu konfiguriert ist, Bilder aus dem Bildgebungsprozess zu verwenden, um eine Position eines Ziels im Körper eines Subjekts zu bestätigen.

14. System nach Anspruch 1, wobei die ersten Informationen Dosisinformationen umfassen, die einen Dosiswert, eine Menge von Dosiswerten oder eine Änderung der Dosis umfassen.

15. System nach Anspruch 1, wobei die ersten Informationen Dosisinformationen umfassen, die eine Metrik umfassen, die eine tatsächliche Echtzeit-In-vivo-Dosis darstellt.

## Revendications

1. Système (10) pour déclencher un processus d'imagerie pendant un processus de radiothérapie, comprenant :
une unité de traitement (54) configurée pour :
obtenir des premières informations ; et
traiter les premières informations pour déterminer si un premier critère prédéfini est satisfait (204, 404) ;
dans lequel l'unité de traitement (54) est configurée pour générer un signal pour entraîner le lancement d'un processus d'imagerie qui comporte une distribution d'énergie d'imagerie sur la base au moins en partie d'un résultat de l'acte de traitement (206), dans lequel les premières informations concernent un état d'un dispositif du processus de traitement (202, 402) ;
**caractérisé en ce que** :
les premières informations comprennent une durée de fonctionnement du dispositif.

2. Système selon la revendication 1, dans lequel les premières informations concernant l'état du dispositif comprennent une ou plusieurs positions de portique, ou une quantité de changement de position de portique.

3. Système selon la revendication 1, dans lequel les premières informations concernant l'état du dispositif comprennent une position de lit ou un changement de position de lit.

4. Système selon la revendication 1, dans lequel les premières informations concernant l'état du dispositif comprennent une configuration de collimateur ou une modification de la configuration de collimateur.

5. Système selon la revendication 1, dans lequel les premières informations concernant l'état du dispositif comprennent une dose totale distribuée, ou une quantité de changement de dose distribuée par le dispositif.

6. Système selon la revendication 1, dans lequel les premières informations concernant l'état du dispositif comprennent le temps, ou une quantité de changement dans le temps, associé à un fonctionnement du dispositif.

7. Système selon la revendication 1, dans lequel les premières informations concernant l'état du dispositif comprennent un signal provenant du dispositif, ou une quantité de changement du signal provenant du dispositif tiers.

8. Système selon la revendication 1, dans lequel les premières informations concernant l'état du dispositif comprennent un signal provenant d'un opérateur du dispositif.

9. Système selon la revendication 1, dans lequel l'unité de traitement (54) est en outre configurée pour :
obtenir des secondes informations (406) ; et
traiter les secondes informations pour déterminer si un second critère prédéfini est satisfait (408) ;
dans lequel l'unité de traitement (54) est configurée pour générer le signal pour entraîner le lancement du processus d'imagerie sur la base au moins en partie du résultat de l'acte de traitement des premières informations et d'un résultat de l'acte de traitement des secondes informations (410).

10. Système selon la revendication 9, dans lequel les secondes informations comprennent des informations de synchronisation ou une position de centrage.

11. Système selon la revendication 1, dans lequel le dispositif comprend au moins l'un d'un portique (12), d'un collimateur (22) et d'un lit (14).

12. Système selon la revendication 1, dans lequel l'unité de traitement (54) est en outre configurée pour recevoir une entrée d'utilisateur indiquant un désir de commencer le processus d'imagerie, dans lequel l'unité de traitement (54) est configurée pour générer le signal pour entraîner le lancement du processus d'imagerie lorsque le résultat de l'acte de traitement indique que le premier critère prédéfini est satisfait et lorsque l'entrée d'utilisateur est reçue par l'unité de traitement (54).

13. Système selon la revendication 1, dans lequel l'unité de traitement (54) est en outre configurée pour utiliser des images provenant du processus d'imagerie afin de confirmer une position d'une cible à l'intérieur du corps d'un sujet.

14. Système selon la revendication 1, dans lequel lesdites premières informations comprennent des informations de dose comprenant une valeur de dose, un ensemble de valeurs de dose ou un changement de dose.

15. Système selon la revendication 1, dans lequel lesdites premières informations comprennent des informations de dose comprenant une mesure qui représente une dose in vivo réelle en temps réel.
